(19) **Europäisches Patentamt / European Patent Office / Office européen des brevets**

(11) **EP 3 515 458 B1**

(12) **EUROPEAN PATENT SPECIFICATION**

(45) Date of publication and mention
of the grant of the patent:
**14.04.2021 Bulletin 2021/15**

(51) Int Cl.:
*A61K 33/04* (2006.01)     *A61K 9/00* (2006.01)
*A61P 1/16* (2006.01)

(21) Application number: **17768471.9**

(86) International application number:
**PCT/EP2017/073878**

(22) Date of filing: **21.09.2017**

(87) International publication number:
**WO 2018/055025 (29.03.2018 Gazette 2018/13)**

(54) **THIOSULFATE FOR TREATING HEPATIC ISCHEMIA/REPERFUSION INJURY**

THIOSULFAT ZUR BEHANDLUNG VON ISCHÄMIE-/REPERFUSIONSSCHÄDEN

THIOSULFATE POUR LE TRAITEMENT DE L'ISCHÉMIE HÉPATIQUE/LÉSIONS DE REPERFUSION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.09.2016 EP 16189948**

(43) Date of publication of application:
**31.07.2019 Bulletin 2019/31**

(73) Proprietor: **Universitätsklinikum Jena
07743 Jena (DE)**

(72) Inventors:
• **SETTMACHER, Utz**
**07743 Jena (DE)**
• **BAUER, Michael**
**07743 Jena (DE)**

(74) Representative: **Schulz Junghans
Patentanwälte PartGmbB
Großbeerenstraße 71
10963 Berlin (DE)**

(56) References cited:
**WO-A2-2008/043081**

• **MARUTANI EIZO ET AL: "Thiosulfate Mediates Cytoprotective Effects of Hydrogen Sulfide Against Neuronal Ischemia.", JOURNAL OF THE AMERICAN HEART ASSOCIATION 06 NOV 2015, vol. 4, no. 11, 6 November 2015 (2015-11-06), XP008183312, ISSN: 2047-9980**
• **S. JHA ET AL: "Hydrogen sulfide attenuates hepatic ischemia-reperfusion injury: role of antioxidant and antiapoptotic signaling", AMERICAN JOURNAL OF PHYSIOLOGY: HEART AND CIRCULATORY PHYSIOLOGY, vol. 295, no. 2, 27 June 2008 (2008-06-27), pages H801-H806, XP055346431, US ISSN: 0363-6135, DOI: 10.1152/ajpheart.00377.2008**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

The present invention relates to the use of a thiosulfate for treating hepatic ischemia/ reperfusion injury.

Liver dysfunction develops as a complication of transplantation or after other states of shock and ischemia and portends poor outcome in the critically ill. Injury under these conditions is primarily associated with hepatocytes in the downstream region of the acinus, i.e. those experiencing physiologically lower oxygen tensions and is traditionally referred to as "pericentral necrosis". Previous work revealed that the molecular mechanisms of injury to pericentral hepatocytes encompass both, necrotic as well as apoptotic mechanisms of injury depending on the degree of ischemic damage and the adequacy of restoration of oxygen supply upon reperfusion. Induction of apoptotic, necrotic or necrapoptotic cell death partially depends on competing and partially on shared pathways. However, there is general agreement on the central role of mitochondria that determine the final path to cellular demise.

Hydrogen sulfide ($H_2S$) has lately received attention beside NO and CO as the third gaseous signalling molecule and like the other inorganic gases shows a striking "hormetic" profile with potential (mito-)protective actions at low, and significant toxicity, e.g. uncoupling of the respiratory chain, at higher concentrations. The resulting narrow therapeutic window of simple $H_2S$ donors, such as sodium hydrogensulphide (NaHS) has limited its use in human disease despite promising preclinical evidence, in particular in models of ischemia and reperfusion (IRI). WO2008043081 discloses the use of hydrogen sulfide in treatment of hepatic ischemia.

$H_2S$ is subject to stepwise oxidation to persulfide, thiosulfate ($S_2O_3^{2-}$), sulfite and sulfate. The intermediate product of $H_2S$ oxidation, thiosulfate is stable in solution, well tolerated by humans even at very high amounts and approved for therapeutic use in a variety of very rare clinical conditions, such as cyanide poisoning or vascular calcifications in uremia. Reports have documented that several grams of STS can be safely administered within minutes and thereby serve as a non-toxic, clinically applicable donor of hydrogen sulphide. Thus, the problem underlying the present invention is to provide compounds suitable for the treatment of hepatic ischemia/reperfusion injury. This problem is solved by the subject-matter of the independent claims.

The surprising finding was made that hepatic ischemia/reperfusion injury may be treated with a thiosulfate such as sodium thiosulfate. Particularly, it was found that the administration of sodium thiosulfate (STS) can serve as a stratagem to achieve mitoprotection observed with donors of $H_2S$, however, with a substantially improved safety profile.

According to a first aspect of the invention, a compound is provided for the use in a method for treating hepatic ischemia/reperfusion injury, wherein the compound is a thiosulfate.

The term "thiosulfate" in the context of the present specification refers to derivatives of the thiosulfuric acid, particularly salts thereof comprising the thiosulfate ion $S_2O_3^{2-}$.

In certain embodiments, the compound is sodium thiosulfate ($Na_2S_2O_3$, CAS No 7772-98-7, CAS No 101202-17-7).

Advantageously, thiosulfate, particularly sodium thiosulfate, is tolerated in high concentrations by the mitochondrial respiratory chain. Moreover, the superior pharmacokinetics and -dynamics of STS as a $H_2S$ donor enable its clinical use.

In certain embodiments, the compound of the invention is administered according to the following dosage regimen:

a) administering an initial dose of the compound of the invention to a patient on the first day of treatment.

The term "first day of treatment" in the context of the present specification refers to 12 to 24 hours of treatment with the compound of the invention, wherein particularly the first day of treatment starts with the administration of the initial dose. The treatment may be started before, during or after a liver transplantation or a tumour resection.

In certain embodiments, the initial dose is administered immediately after or less than 30 min, 1 h or 2 hours after positive diagnosis of a haemorrhagic shock in said patient, or immediately after or less than 30 min, 1 h or 2 hours after conducting a liver transplantation or a tumour resection.

In certain embodiments, the donor liver for transplantation is characterized by a preimpairment or pre-existing damage.

In certain embodiments, the donor liver for transplantation originates from a marginal donor, particularly a donor with a significant risk for impaired post-transplant function such as an aged donor.

In certain embodiments, the donor liver for transplantation is a fatty liver.

In certain embodiments, the initial dose comprises the compound of the invention, particularly sodium thiosulfate, in the range of 0.1 mg per kg body weight of the patient to 500 mg per kg body weight of the patient. In certain embodiments, the initial dose comprises the compound of the invention, particularly sodium thiosulfate, in the range of 1 mg per kg body weight of the patient to 50 mg per kg body weight of the patient. In certain embodiments, the initial dose comprises the compound of the invention, particularly sodium thiosulfate, in the range of 3 mg per kg body weight of the patient to 45 mg per kg body weight of the patient. In certain embodiments, the initial dose comprises the compound of the invention, particularly sodium thiosulfate, in the range of 30 mg per kg body weight of the patient to 450 mg per kg body weight of the patient. In certain embodiments, the compound of the invention is orally administered. In certain embodiments, the compound of the invention is administered intraperitoneally. In certain embodiments, the compound of the invention is administered subcutaneously. In certain embodiments, the compound of the invention is administered intravenously.

According to another aspect of the invention, a pharmaceutical composition for use in a method for treating hepatic ischemia/reperfusion injury is provided. The pharmaceutical composition comprises the compound of the invention, particularly sodium thiosulfate.

The pharmaceutical compositions may be formulated for enteral administration, such as nasal, buccal, rectal or, especially, oral administration, and for parenteral administration, such as subcutaneous, intravenous, intrahepatic or intramuscular administration. The pharmaceutical compositions comprise from approximately 1% to approximately 95% active ingredient, preferably from approximately 20% to approximately 90% active ingredient.

Similarly, a dosage form for the treating ischemia/reperfusion injury is provided, comprising the compound of the invention, particularly sodium thiosulfate. Dosage forms may be for enteral administration, such as nasal, buccal, rectal, transdermal or oral administration, or as an inhalation form or suppository. Alternatively, parenteral administration may be used, such as subcutaneous, intravenous, intrahepatic or intramuscular injection forms. Optionally, a pharmaceutically acceptable carrier and/or excipient may be present.

In certain embodiments, the compound of the invention, particularly sodium thiosulfate, is applied as an oral formulation, nasal inhalant, injection form, intravenous form, suppository or topical formulation, wherein an oral formulation or an intravenous form is preferred.

In certain embodiments, the dosage form comprises the compound of the invention, particularly sodium thiosulfate, in the range of 0.1 mg per kg body weight of the patient to 500 mg per kg body weight of the patient. In certain embodiments, the dosage form comprises the compound of the invention, particularly sodium thiosulfate, in the range of 1 mg per kg body weight of the patient to 50 mg per kg body weight of the patient. In certain embodiments, the dosage form comprises the compound of the invention, particularly sodium thiosulfate, in the range of 3 mg per kg body weight of the patient to 45 mg per kg body weight of the patient. In certain embodiments, the dosage form comprises the compound of the invention, particularly sodium thiosulfate, in the range of 30 mg per kg body weight of the patient to 450 mg per kg body weight of the patient.

The invention is further illustrated by the following examples and figures, from which further embodiments and advantages can be drawn. These examples are meant to illustrate the invention but not to limit its scope.

## Description of the figures

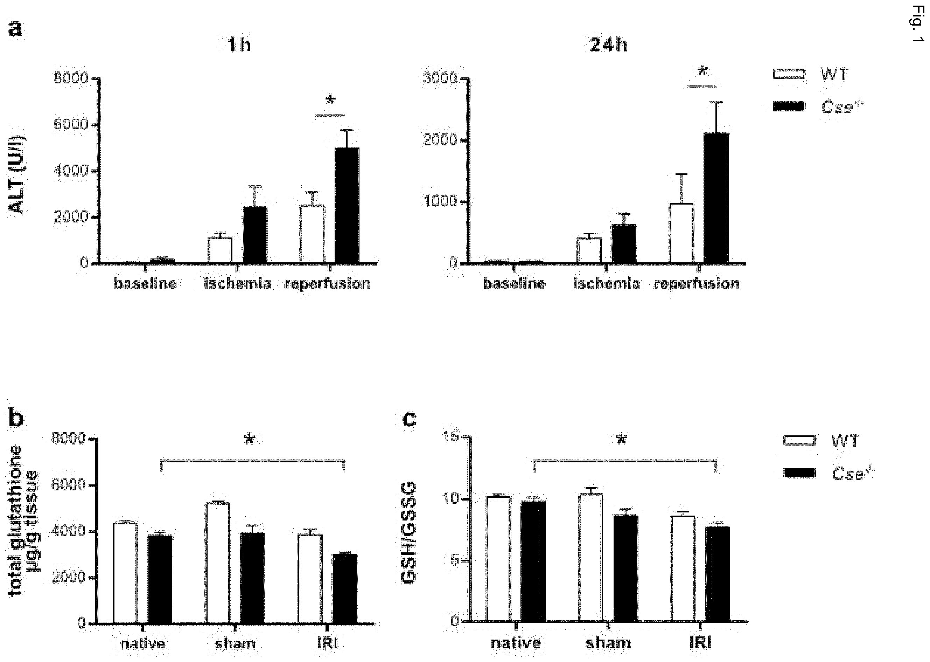

Fig. 1　shows that endogenous $H_2S$ protects against tissue injury in hepatic IRI model. (a) WT and $Cse^{-/-}$ mice underwent 60 min of ischemia of the left lateral lobe liver lobe followed by 1 h or 24 h of reperfusion. Baseline plasma samples were generated 7 days before the experiment. Plasma ALT levels in U/l. *$P < 0.05$, by Two-way ANOVA with Sidak's multi comparison test. n=5-8/group. Data shown in mean + SEM. **(b)** Total glutathione levels measured after 1 h reperfusion in liver tissue. (c) GSH/GSSG ratio calculated to determine the antioxidant capacity in the liver tissue. *$P < 0.05$, by Two-way ANOVA with Sidak multi comparison test. n=6-8/group. Data shown as mean + SEM.

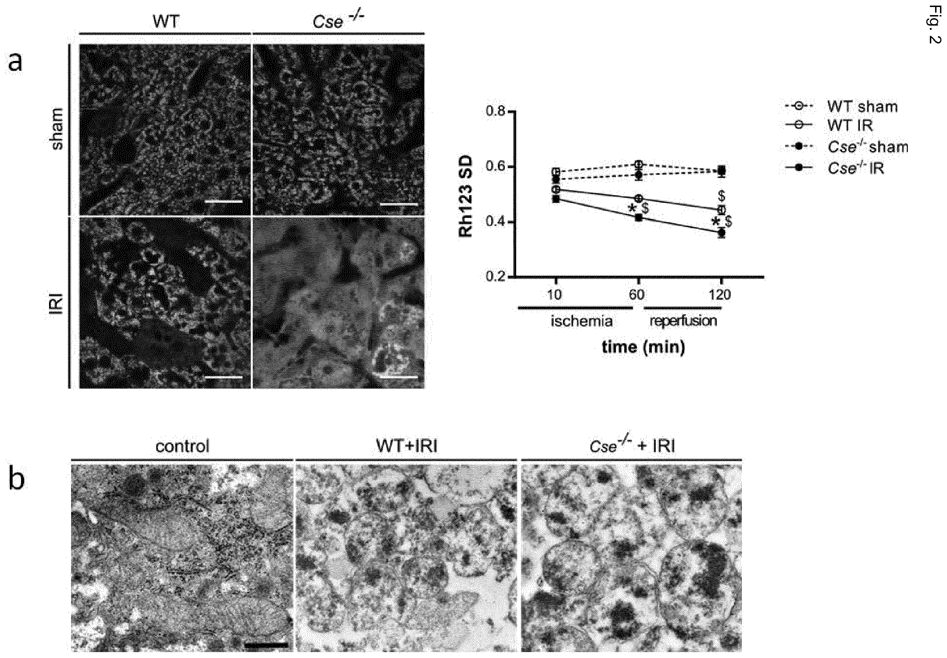

Fig. 2　shows the protective effects of endogenous $H_2S$ involve mitochondria. **(a)** Rhodamine 123 was injected into the tail vein 5 min before the onset of 1 h liver ischemia and intravital microscopy was performed. After controlled ischemia time, the clamped lobe was reperfused for 1 h. Representative images are shown. Left, WT; right, $Cse^{-/-}$; top, sham operation; bottom, ischemia-reperfusion. Scale bar is 30 $\mu$m. Rhodamine 123 standard deviation was calculated to evaluate the depolarisation of the mitochondria. *$P < 0.05$, by Two-way ANOVA with Tukey's multi comparison test between WT and $Cse^{-/-}$, $^\$P < 0.05$, by Two-way ANOVA with Tukey's multi comparison test to 10 min, n=3-6/group. Data shown as mean $\pm$ SEM. **(b)** Morphological ultrastructure of hepatic mitochondria by transmission electron microscopy. left, healthy control; center, WT+IRI, right, $Cse^{-/-}$+ IRI. Scale bar is 500 nm.

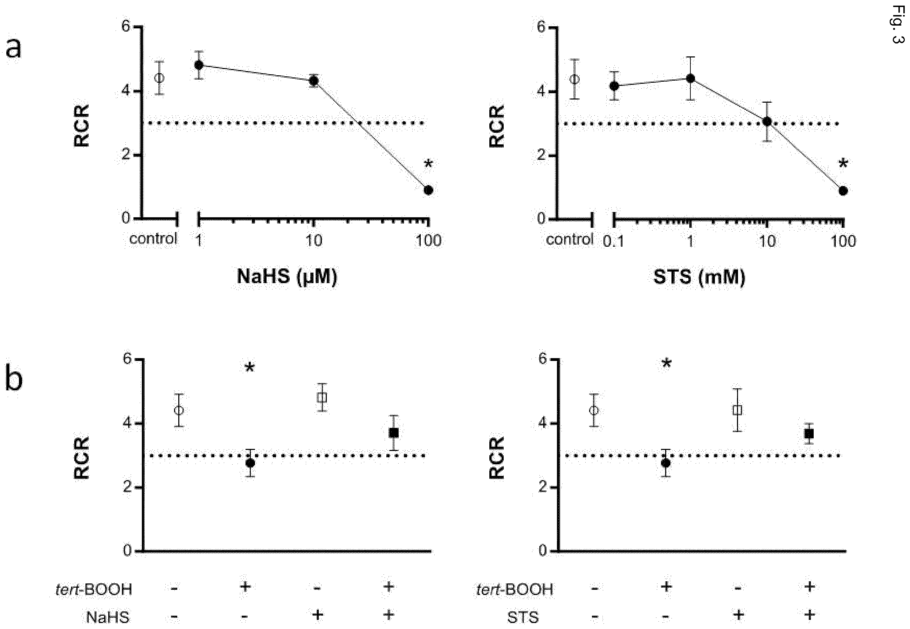

Fig. 3　shows the therapeutic potential of $H_2S$ donors. RCR was calculated by the ratio of state III (ADP-dependent respiration) and state IV (ADP-independent respiration). **(a)** left: 0-100 $\mu$M NaHS; right: 0-100 mM STS. **(b)** left: RCR of control, 200 $\mu$M tert-BOOH, 1 $\mu$M NaHS and 200 $\mu$M tert-BOOH + 1 $\mu$M NaHS. right: RCR of control, 200 $\mu$M tert-BOOH, 1 $\mu$M NaHS and 200 $\mu$M tert-BOOH + 1 $\mu$M STS. *$P < 0.05$, by ANOVA with Dunnett's multiple comparisons test. n=4-6/group. Data shown as mean $\pm$ SD.

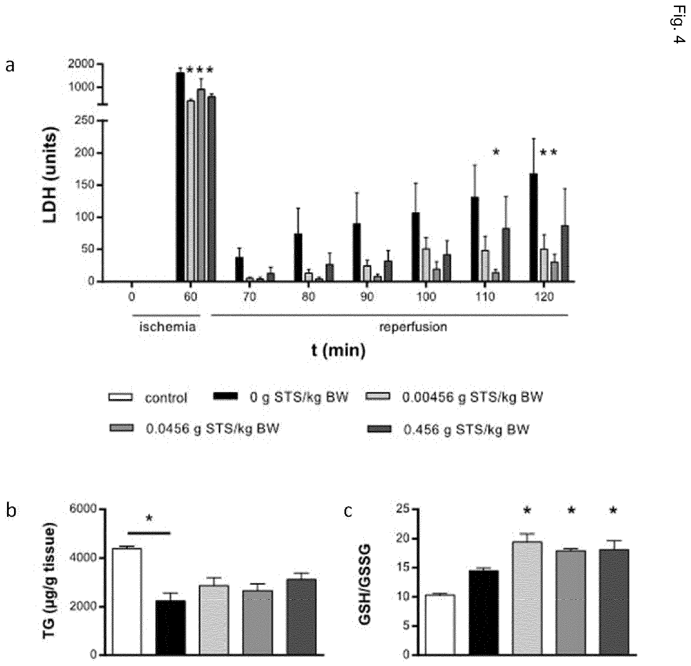

Fig. 4    shows that STS protects against hepatic IRI. Isolated liver perfusion with IRI, at the onset of reperfusion the KHB were charged with 0.0045 g STS/kg BW, 0.045 g STS/kg BW, 0.45 g STS/kg BW or without STS (w/o STS). Controls were perfused with KBH without IRI. **(a)** LDH level were measured in the hepatic outflow. *P < 0.05, by Two-way ANOVA with Sidak multi comparison test every time point was tested against the control group without STS. n=4-8/group. Data shown as mean + SEM. **(b)** Total glutathione. **(c)** GSH/GSSG ratio. *P < 0.05, by Kruskal-Wallis with Dunn's multi comparison test. n=4-8/group. Data shown as mean $\pm$ SEM.

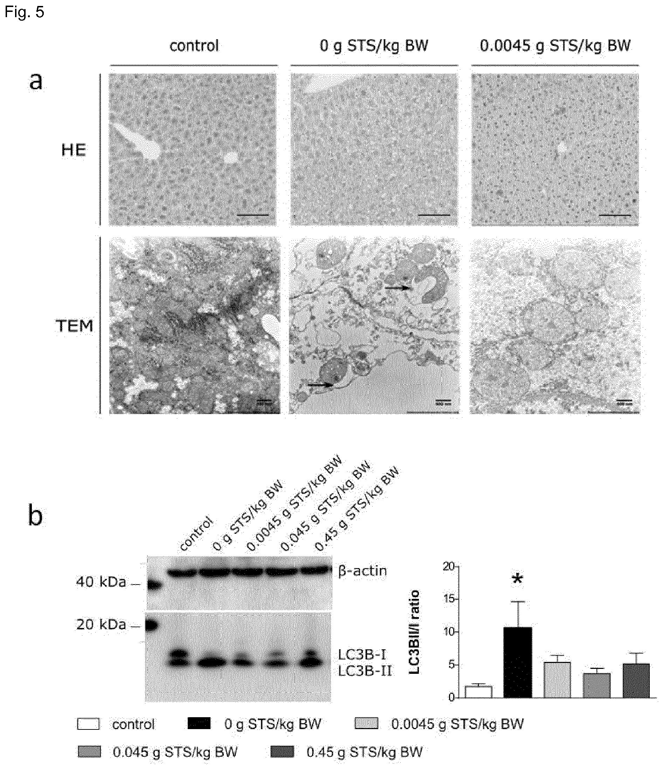

Fig. 5    shows that STS impacts mitophagy. Molecular Biological analysis of isolated liver perfusion samples. **(a)** Top, HE staining of liver sections. Representative image for healthy control, IRI - STS and IRI + STS. Scale bar, 100 $\mu$m. Bottom, Representative transmission electron microscopy sections from healthy controls, IRI - STS and IRI + STS treated livers, 1 h after reperfusion. Arrows indicating space between outer and inner mitochondrial membrane. Scale bar, 500 nm. **(b)** Representative western blots for the analysed target proteins LC3B-I and LC3B-II. $\beta$-actin serves as a loading control. Densitometric analysis of Western blots. *P < 0.05, by Kruskal-Wallis with Dunn's multi comparison test. n=4-8/group. Data shown as mean $\pm$ SEM.

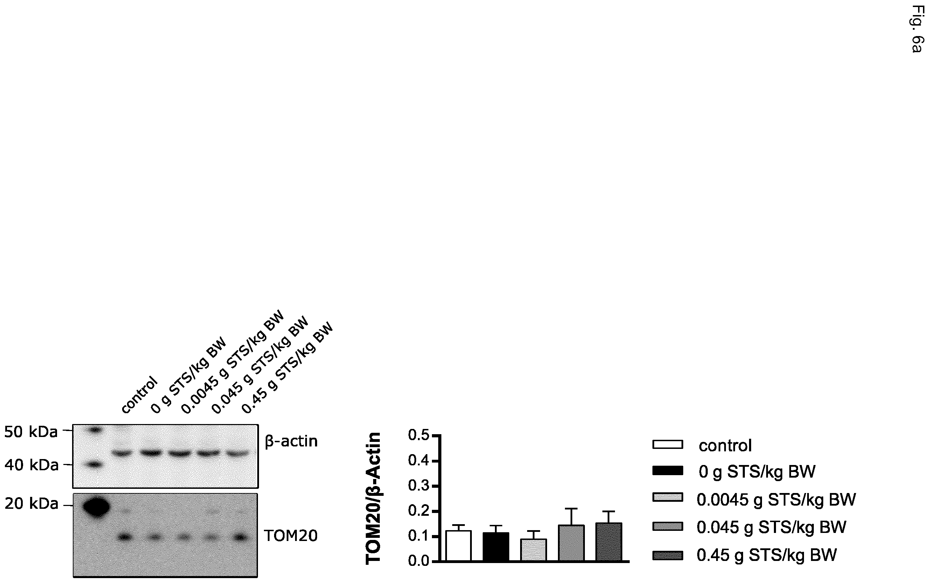

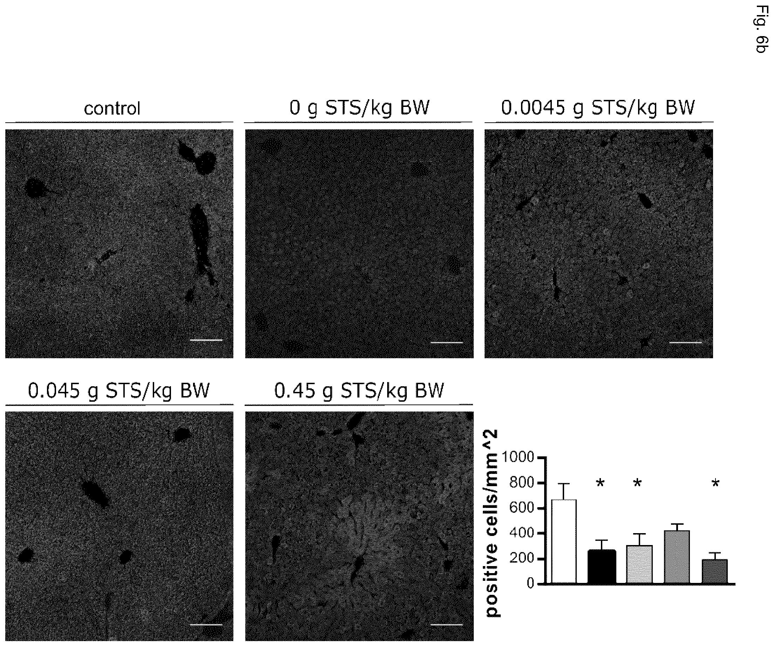

Fig. 6    shows that STS protects mitochondrial membrane protein expression. **(a)** Representative western blots TOM20. $\beta$-actin serves as a loading control. Densiometric analysis of western blots. **(b)** Immunostaining of TOM20. Scale bar is 100 $\mu$m. Positive stained cells/mm$^2$ as mean + SEM. *P < 0.05, by One-way ANOVA Dunnett's multi comparison test. n=4-8/group. Data shown as mean + SEM.

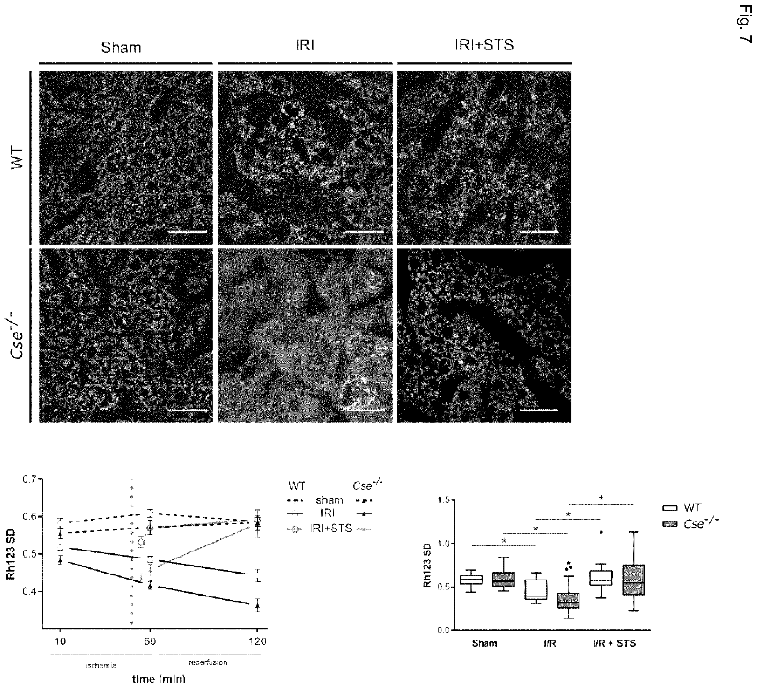

Fig. 7    shows that STS protects against mitochondria depolarisation *in vivo*. Rhodamine 123 was injected into the tail vein 5 min before the onset of 1 h liver ischemia and IVM was performed. Saline or 0.045 g STS/kg BW was injected intravenously 1 min prior to the onset of liver reperfusion (1 h). Representative images are shown. Top, WT; bottom, *Cse*[-/-]. Left, sham operation; center, ischemia-reperfusion; right, IRI with STS application. Scale bar is 30 $\mu$m. SD of the Rhodamine 123 fluorescence signal over time. *P < 0.05, by two-way ANOVA with Tukey's multiple comparison test of IRI with vs without STS treatment, *P < 0.05, by two-way ANOVA with Tukey's multiple comparison test to 10 min, n=3-6/group. Data shown as mean $\pm$ SEM. SD of the Rhodamine 123 fluorescence signal at 120 min. *P < 0.05, by Kruskal-Wallis with Dunn's multiple comparison test. n=3-6/group. Data shown as box plots with whiskers with Tukey.

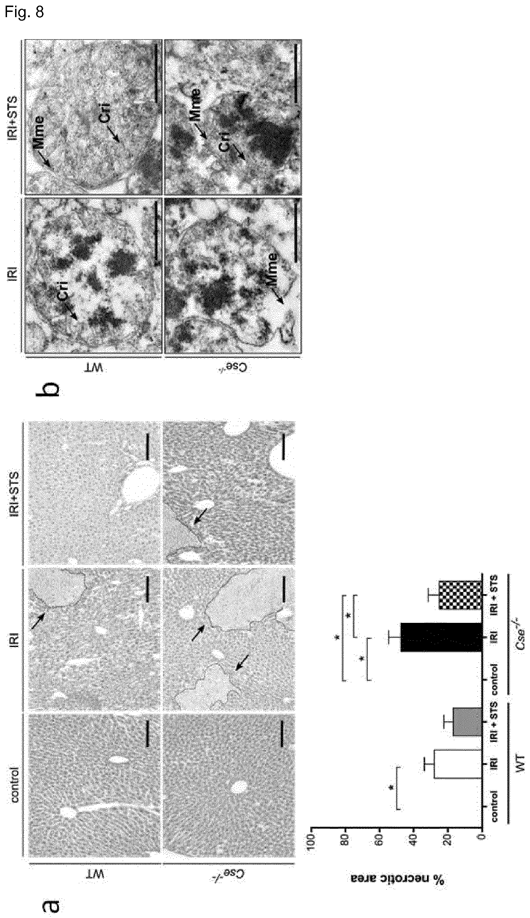

Fig. 8    shows that STS protects against necrosis *in vivo*. **(a)** Saline or 0.045 g STS/ kg BW was injected intravenously 1 min prior to the onset of liver reperfusion. Mice underwent 60 min of ischemia followed by 24 h of reperfusion. Representative images of HE staining. Top, WT; bottom, *Cse*[-/-]. Left, sham operation; center, ischemia-reperfusion; right, ischemia-reperfusion with STS application. Necrotic areas are indicated with an arrow. Scale bar is 100 $\mu$m. Relative necrotic areas based on Yao et al. (2014). *P < 0.05, by Kruskal-Wallis with Dunn's multiple comparison test. n=4-5/group. Data shown as mean with SEM. **(b)** Morphological ultrastructure of mitochondria by transmission electron microscopy. **Mme** = Membrana mitochondrialis externa **Cri** = Crista mitochondrialis. Top, WT; bottom, *Cse*[-/-]. left, ischemia-reperfusion; right, ischemia-reperfusion with STS application. Scale bar is 500 nm.

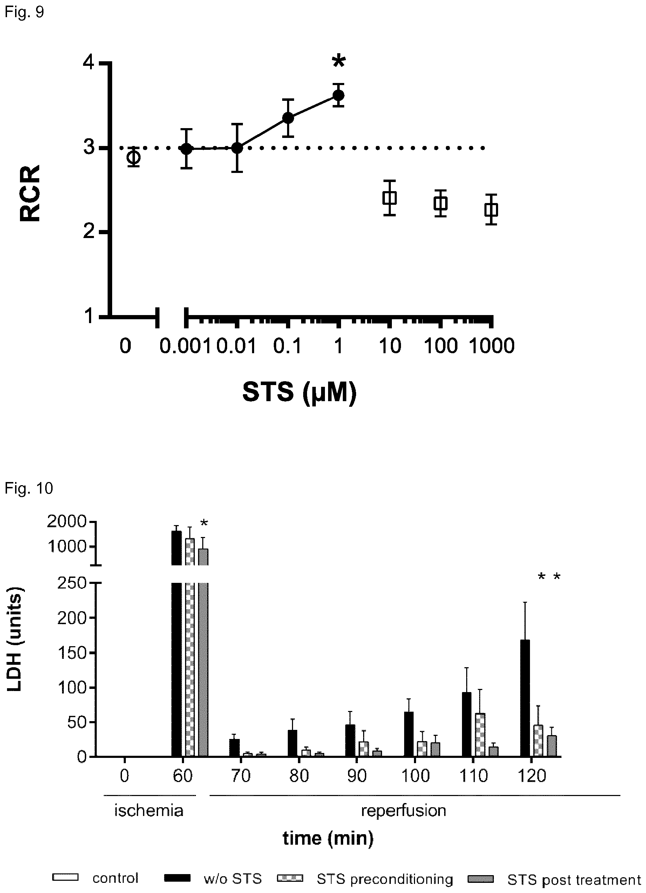

Fig. 9    shows the dose-response relationship of STS under *tert-BOOH* stress. RCR was calculated by the ratio of state III (ADP-dependent respiration) and state IV (ADP-independent respiration). Mitochondria were challenged by 200 $\mu$M *tert-BOOH* in presence of 0.001 to 1000 $\mu$M STS. *P < 0.05, by Kruskal-Wallis with Dunn's multi comparison test. n=4-6/group. Data shown as mean $\pm$ SD.

Fig. 10   shows the STS application time point in *ex vivo* IRI model. Either at the beginning of perfusion or at start of reperfusion 0.045 g STS/kg BW were applied. LDH level were measured in the hepatic outflow. *P < 0.05, by Two-way ANOVA with Sidak multi comparison test every time point was tested against the control group without STS. n=4-8/group. Data shown in mean $\pm$ SEM.

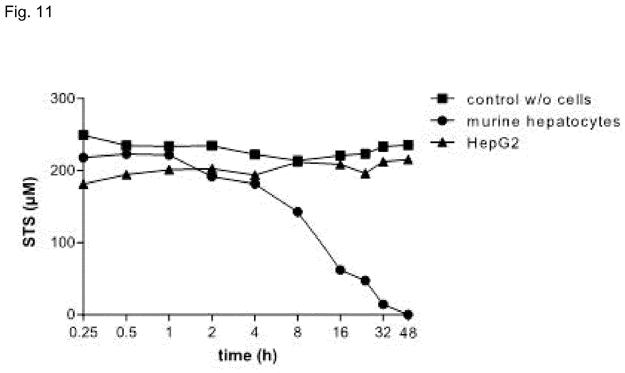

Fig. 11   shows that primary murine hepatocytes metabolise STS. Primary murine hepatocytes and HepG2 cells were incubated with 200 $\mu$M STS for 48 h. Medium without (w/o) cells served as control. STS concentration was measured in the supernatant. Data shown as mean.

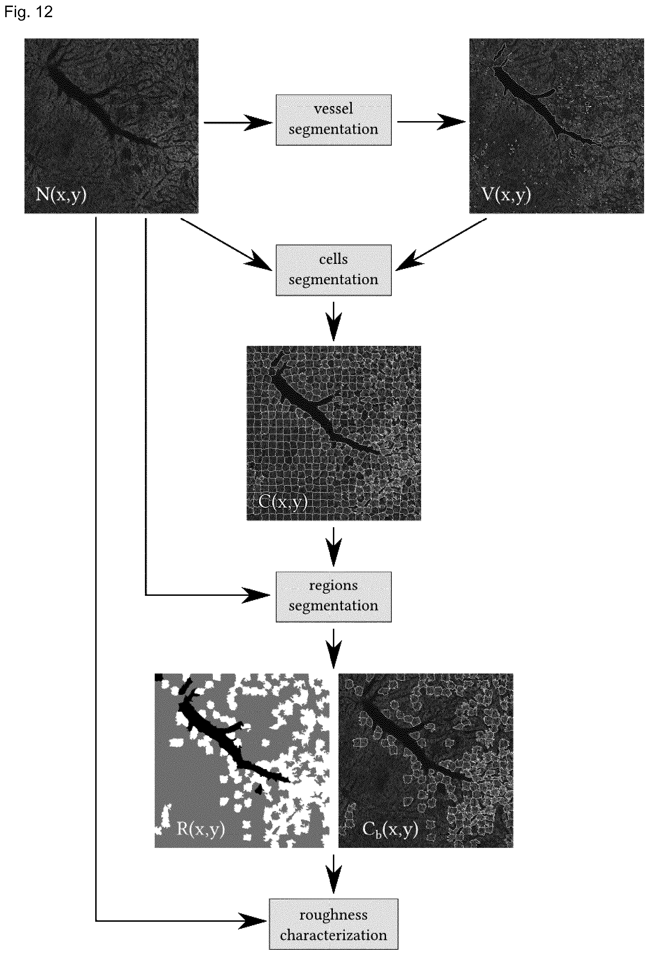

Fig. 12   shows the schematic workflow of the automated image analysis.

**Examples**

Sodium thiosulfate is tolerated at high concentrations by the mitochondrial respiratory chain. Furthermore, it could be shown that sodium thiosulfate protectively acts on the mitochondrial respiratory chain by capturing the reactive oxygen species. Additionally, sodium thiosulfate reduces the secretion of lactate dehydrogenase (LDH) in an *ex vivo* model for isolated liver perfusion. This effect correlates with a smaller autophagy und the maintenance of the mitochondrial fine structure. Particularly, sodium thiosulfate stabilizes the mitochondrial structure and function even in severely damaged tissues.

*In vivo* hepatic IRI results in a depolarisation of the mitochondria, which could be verified by intra vital microscopy. This depolarisation manifests in a decreased granularity of the mitochondrial network. Treatment with sodium thiosulfate before the occurrence of the reperfusion leads to a stabilization of the mitochondrial network, whereby the energy balance of the liver can be maintained.

To sum up, the present invention provides evidences that the use of thiosulfate, particularly sodium thiosulfate, in the IRI model reduces the liver damage. Thereby, novel therapies for liver transplantations or treatment of haemorrhagic shock are provided. Particularly, the application of the limited resources in term of human donor livers can be extended to marginal grafts, hence such one being characterized by preimpairments, for example, livers of aged donors or fatty livers. Additionally, the loss of organs with the primary non-function can be reduced.

**Endogenous $H_2S$ protects against tissue damage**

To investigate the role of $H_2S$ in IRI induced tissue damage a comparative analysis of wild type mice (WT) and a cystathionine γ-lyase deficient strain ($Cse^{-/-}$) was performed. Cystathionine γ-lyase (CSE) is known a key mediator of endogenous $H_2S$ production in mammalian tissue. The data reveal a significant increase in the alanine aminotransferase (ALT) levels after 1 h ischemia followed by 1 h or 24 h reperfusion in the $Cse^{-/-}$ group suggesting a protective function of $H_2S$ in hepatic IRI (Fig. 1a).

Next, the antioxidant capacity of livers was analysed 1 h after reperfusion. The total glutathione levels and the GSH/GSSG ratio were assayed in liver tissue of mice challenged by IRI. Native animals of both genotypes were used for control. In addition, animals were sham operated to investigate the influence of the surgery on the antioxidant capacity. As shown in Fig. 1b and 1c both the level of total glutathione as well as the ratio of GSH and its oxidized form GSSG significantly decreased in livers 1 h after reperfusion of $Cse^{-/-}$ mice. In contrast, WT animals exhibited no differences of both parameters before and after IRI. These data suggest an important function of CSE mediated $H_2S$ in the maintenance of antioxidant capacity of the liver.

To further explore the role of $H_2S$ in hepatic IRI, the integrity of mitochondria as the key organelles of redox processes was investigated in liver cells. Intravital microscopy has been used to monitor the mitochondria network in the used hepatic injury model. The cationic fluorophore Rhodamine 123 was injected to visualize the mitochondria in hepathocytes. Fig. 2a presents recordings of sham and IRI-challenged livers suggesting decreased granularity of the mitochondria network during the ischemia reperfusion process.

Quantification of the fluorescence signals in the accompanying diagram indicates acceleration of IRI induced decrease of mitochondrial granularity in $Cse^{-/-}$ mice in comparison to WT. In CSE deficient mice the granularity of the mitochondria network declined further until the end of the experiment. Further analysis revealed higher number of polarized mitochondria in liver tissue of WT mice compared to the $Cse^{-/-}$ group after ischemia and reperfusion.

Together these data indicate a protective function of $H_2S$ during hepatic IRI. Suppression of mitochondrial disintegration by $H_2S$ seems to substantially contribute to the less pronounced damaging effects of IRI in liver of WT mice.

**Influence of NaHS and STS on mitochondrial respiration chain**

To mimic the hypothesized protective effects of $H_2S$ by pharmacological means, sodium hydrogen sulphide (NaHS) was used as a fast-releasing $H_2S$ donor as well as sodium thiosulfate (STS). As shown in Fig. 3a both NaHS and STS stimulated mitochondrial respiratory activity at low doses, but whereas 100 μM of NaHS already induced inhibitory effects on respiratory control ratio (RCR), STS was tolerated up to 10 mM.

Under oxidative stress, mimicked by application of *tert*-butyl hydroperoxide (*tert*-BOOH), both substances exhibited a protective effect on RCR in the concentration range of 1 μM (Fig. 3b). Higher concentrations of STS inhibited the mitochondrial respiration chain and decreased RCR (Fig. 9). These data further underline protective capacity of $H_2S$ in mitochondrial dysfunction. STS has been disclosed as an efficient mediator of RCR preservation.

**STS protects the liver from ischemia-reperfusion injury - ex vivo**

Due to the protective effects of STS on mitochondrial respiration, more widespread effects of the salt on liver tissue challenged by IRI were explored. IRI induced hepatic dysfunction is associated with disturbed global and microcirculatory liver perfusion. To implement the ex *vivo* IRI model, the liver was perfused via the portal vein with oxygenated Krebs-Henseleit buffer for 5 min one-way and 5 min in reverse. Afterwards, the pump was stopped for 1 h followed by 1 h reperfusion. During reperfusion, tissue samples were collected every 10 min. To investigate the effects of STS on IRI, the salt was applied in three doses to the perfused liver. 0.0045 g STS/kg BW was defined as low dose, 0.045 g STS/kg BW as medium dose and 0.45 g STS/kg BW as high dose. The initial dose of 0.45 g STS/kg BW (HD) was chosen based on a recent study showing protective effects of STS on renal function in hyperoxaluric rats. In one animal group, livers were only perfused with buffer solution, but without STS. Another group was perfused without stopping the pump to determine the injury by the extracorporeal asanguineous perfusion itself. LDH levels were measured in the outflow samples to quantify tissue injury.

As shown in Fig. 4a, the low and medium doses of STS reduced LDH levels significantly at 60, 110 and 120 min reperfusion. In contrast, high dose of STS only reduced LDH levels after 60 min and 120 min. Hence, the medium dose of STS exhibited superior effect in our model. Application of this STS dose reduced the LDH level by 82,3 % in comparison to the group without STS at 120 min reperfusion (Fig. 4a). Ten times reduction of STS dose exhibited a similar effect decreasing the LDH levels by 81.9 %. Interestingly, high dose of STS induced only 48 % reduction of LDH level. Together these data suggest an exclusive therapeutic potential of STS compared to NaHS.

To explore the STS mode of action in the model, the total glutathione levels and also the ratio of reduced and oxidized glutathione species (GSH/GSSG) were analysed. The amount of total glutathione was significantly decreased by IRI independently on STS treatment (Fig. 4b). In contrast, all applied doses of STS induced a significant increase of the GSH/GSSG ratio after 1 h ischemia and 1 h reperfusion (Fig. 4c).

To optimize the application time point, the most efficient medium dose of STS was chosen to investigate time-dependent effects on LDH release. STS was applied either at the beginning of the experiment (pre-treatment group) or at the onset of reperfusion (post-treatment group). Control experiments without ischemia and reperfusion and with IRI in the absence of STS were also conducted. Fig. 10 reveals the reduction of LDH levels after 120 min pre-treatment, but not after 60 min. In contrast, the post-treatment group exhibited reduced LDH levels both at, 60 min and 120 min. The pre-treatment group showed a reduction of 56.4 % IRI induced LDH level compared to 82.3 % in the post-treatment group.

Together these data indicate that the treatment of mouse liver with clinically achievable doses of STS at the onset of reperfusion leads to a substantial protection of the mitochondrial function and corresponding decrease of IRI induced tissue damage.

**STS protects against mitochondrial dysfunction - ex vivo**

To further explore the protective effects of STS on mitochondria, liver tissue was analysed by transmission electron microscopy after hepatic IRI in the absence of STS or after treatment with low dose STS. As shown in Fig. 5a (bottom) IRI induced significant structural changes of mitochondria including detachment of the inner mitochondrial membrane from the outer membrane (arrows).

These results suggest IRI induced collapse of the mitochondrial architecture and consequent organelle failure. In contrast, in the STS treated group the mitochondria retained their normal structure.

The intriguing protective effect of STS on the mitochondrial structure during IRI urged exploration of the mechanistic background. Autophagy of mitochondria (mitophagy) has been described as a key process involved in mitochondrial turnover and. First, the main autophagy marker, LC3B-I and LC3B-II were investigated by Western blot analysis in liver tissue 1 h after reperfusion. Representative blots for LC3B-I, LC3B-II are shown in Fig. 5b. To quantify the level of mitophagy, the ratio between LC3B-II and LC3B-I was calculated. The group with no STS treatment, which underwent IRI, however, showed a significant increase in the ratio from 1.7 (healthy control) to 10.6, whereas in all three STS treated groups a significantly smaller increase was observed (5.4 at low dose; 3.6 at medium dose and 5.1 at high dose). The data indicate a protective action of STS on mitochondrial integrity.

In addition to autophagy marker proteins, the translocase of the mitochondrial outer membrane 20 (TOM20) was analysed. TOM20 is responsible for the recognition and translocation of mitochondrial pre-proteins synthesized in the cytosol. Western blot analysis revealed no significant differences of the TOM20 levels between healthy controls and IRI treated livers as well as in the STS treated groups (Fig. 6a). To detect a possible specific loss of TOM20 in liver cells, immunofluorescence staining was carried out. In fig. 6b representative tissue slides stained for TOM20 are presented. The healthy control group showed an overall homogenous expression of TOM20 (Fig. 6b). In contrast, a decrease of the TOM20 signal was detected in livers affected by IRI without STS treatment. STS at low and high dose did not significantly increase TOM20 signal. Around the central veins, TOM20 was still expressed whereas the signal decreased with expanding distance. Following quantification of positive cells per $mm^2$, the TOM20 signal was significantly decreased in the groups without STS (258 positive cells per $mm^2$), low (305 positive cells per $mm^2$) and high dose (194 positive cells per $mm^2$), compared to the healthy control with 672 positive cells per $mm^2$. In contrast, medium dose of STS precluded significant decrease in the TOM20 signal (422 positive cells per mm$^2$) again indicating protective function of STS on mitochondrial functional pattern.

**STS rescues livers form IRI *in vivo***

Finally, the effect of STS on hepatic IRI was investigated *in vivo.* Intravital microscopy has been used to analyse the early reperfusion injury in WT and *Cse$^{-/-}$* mice. Assay of plasma parameters and histological staining was applied to analyse later IRI. Rhodamine 123 was injected into the tail vein 5 min before the onset of the experiment. Subsequently, hepatic IRI was conducted with and without application of medium dose of STS into the tail vein 10 min before onset of reperfusion. The mice were monitored for 2 h without IRI in the sham control group. In fig. 7 representative images are presented. It was observed that the mitochondrial granularity decreased in all animals challenged by hepatic IRI (Fig. 7). STS treatment led to a pronounced recovery of the mitochondrial granularity in the WT group during the beginning of the reperfusion (60 min). This effect was also detected at the end of reperfusion (120 min) for both genotypes.

In a further experiment, the mice underwent 24 h reperfusion to analyse necrosis formation. The tissue injury was investigated by quantifying the necrotic areas in liver tissue sections stained with hematoxylin and eosin (HE). The quantification was based on the score described by Yao et al. (Establishment of a rat model of portal vein ligation combined with in situ splitting. PLoS One, 9 (8): e105511, 2014). Fig. 8a presents the relative necrotic areas. No necrotic areas could be identified in the healthy control groups of both genotypes. IRI induced an increase of necrosis in the WT as well as in the *Cse$^{-/-}$* group. *Cse$^{-/-}$* mice exhibited a significant reduction of the necrotic areas from 47 % to 25 % after treatment with medium dose of STS (Fig. 8a). This effect was not significant in the WT animals. However, also a reduction by trend from 28 % to 17 % was detected, underlining the potential therapeutic value of STS for the treatment of hepatic IRI. The ultrastructure analysis by transmission electron microscopy showed a loss of the mitochondrial membrane integrity in the *Cse$^{-/-}$* group, which underwent IRI (Fig. 8b). In the WT, in the *Cse$^{-/-}$* as well as in the STS treated *Cse$^{-/-}$* group an electron-dense accumulation was observed, whereas the WT group after STS treatment showed a normal mitochondrial architecture.

**Conclusion**

The data presented here strongly indicate a protective function of H$_2$S in hepatic IRI in mice.

To explore the therapeutic potential of these insights, it was next tried to elevate the H$_2$S level in mitochondria isolated from liver cells by exogenous addition of sulphur salts. NaHS was added and the respiratory control ratio (RCR) was assayed as a measure for functional integrity of mitochondria. NaHS compensated the decline of RCR induced by *tert-BOOH* oxidative stress suggesting protective power at the given dose. Suprisingly, similar protective effects have been shown by STS in the concentration range 0.1 to 1 $\mu$M. NaHS is currently the most widely used tool to generate H$_2$S, but it expresses pronounced lability leading to vast release of H$_2$S if applied to tissue at physiological pH. Immediate and strong increase of H$_2$S may cause rapid fall of blood pressure with detrimental consequences for mammalian organisms. Due to the low stability of NaHS the effects of H$_2$S produced by applying this salt are also short-lived thus impeding therapeutic use of this compound. In contrast to NaHS, STS is quite stable allowing enduring effects on the tissue affected. The relative stability of STS and the tight interconnection of H$_2$S and STS metabolism in liver cells is attested by the herein presented investigation of degradation dynamics of STS in murine hepatocytes (Fig. 11). Thus, the comparative consideration of NaHS and STS as potential mimetics for the protective effects of H$_2$S on IRI induced liver damage clearly supports STS as a treatment opportunity.

Accordingly, the STS effects on IRI induced liver damage was investigated. Experiments have been started using an *ex vivo* liver perfusion model. The dynamics of LDH release during the ischemia reperfusion procedure shown in Fig. 4 demonstrates increasing damage of liver tissue in the untreated liver tissue. STS partially prevents this damaging effect does dependently. Intriguingly, strongest protective effect has been observed at medium STS dose (0,045 g/kg BW) possibly indicating "hormetic" adaptation of the liver to the salt. Highest protective effects of low and medium STS doses were also observed in terms of two parameters indicating the functional integrity of mitochondria: autophagy and expression of the translocase Tom20.

Autophagy has been characterized as a quality control and maintenance process, which is normally provoked by functional impairment of intracellular organelles. In the model presented herein, IRI induced tissue damaged is accompanied by significant increase of autophagy (Fig. 5, LC3 II/I ratio) and low and medium doses of STS have been shown to decrease autophagy. In the context with the high damaging effects of IRI shown in Fig. 4 (LDH release) we interpret these data as an indication for STS induced "optimization" of IRI dependent autophagy, which allows the liver cells on one hand to regenerate malfunctioning mitochondria but on the other hand to prevent supercritical autophagy. Excess autophagy has been described as transition state to cellular apoptosis.

Distinct protective effects of medium and low doses of STS on IRI induced damage of liver cell integrity are fully supported by the investigations of the mitochondrial outer membrane translocase TOM20 presented herein. TOM20 has been shown to recognize and translocate mitochondrial pre-proteins, which have been synthesized in the cytosol. Prevention of the ischemia-induced decrease in mitochondrial Tom20 content by ischemic preconditioning was recently shown to contribute to improved mitochondrial function after IRI. Thus expression of TOM20 could be taken as another measure of functional integrity of mitochondria in liver cells. In our hand IRI induced pronounced decrease of TOM20 content, which could be attenuated by STS indicating another protective effect of the salt.

The STS effects on hepatic IRI were tested in an *in vivo* mouse model. Ischemia was induced in the left lateral lobe of the liver by clamping the hepatic artery and the portal vein and IRI induced damaging effects in liver tissue of WT and *Cse*$^{-/-}$ mice was monitored by different staining techniques and electron microscopy. The comparative analysis of untreated mice and animals treated with STS clearly revealed protective effects of the salt on IRI induced damage.

In conclusion, the investigations presented herein confirm protective action of $H_2S$ in hepatic damage induced by IRI. STS treatment has been proved as a novel approach for the treatment of liver impairment during reperfusion.

## Methods

### Hepatic ischemia-reperfusion

Animal experiments were approved by the local regulatory board (Thuringian State Office for Consumer Protection and Food Safety). *Cse*$^{-/-}$ mice were generated according to Wang et al. (H2S as a physiologic vasorelaxant: hypertension in mice with deletion of cystathionine gamma-lyase. Science, 322 (5901): 587-590, Oct 2008.). The mice were imported by embryonic transfer into the animal facility of the Jena University Hospital and conducted under pathogen free conditions. The original mixed Sv129/C57 background was backcrossed to a 99 % C57BL/6 background. In all experiments male WT and *Cse*$^{-/-}$ mice between 10 and 12 weeks were used for a standardised hepatic IRI model. One week before the experiments started, 100 $\mu$l blood was taken from the orbital sinus. Body temperature was maintained with a heating plate. During the actual experiment, ischemia was induced in the left lateral lobe of the liver by clamping the hepatic artery and the portal vein using a microaneurysm clamp (FST, Heidelberg, Germany). This procedure results in a segmental (30 %) ischemia. After 1 h, the clamp was removed to initiate the reperfusion. The reperfusion time points were set to 1 h or 24 h. Subsequently, the mice were sacrificed and both, plasma and organs, were harvested. During deep isoflurane (Forene®, Abb-Vie Deutschland GmbH & Co. KG, Ludwigshafen, Germany) anaesthesia, the blood was collected and plasma was generated. Next, the liver, the kidney and the spleen were removed and washed with saline.

### Clinical biochemical parameters

The tissue injury parameters were analysed in the generated plasma samples. Therefore, the Fuji Dri-Chem 3500i analyser (Fujifilm, Düsseldorf, Germany) was used to determine the level of circulating alanine aminotransferase (ALT). The analysis was carried out according to the manufactures protocol using parameter specific slides.

### Total glutathione measurement

The measurement of total glutathione as well as the measurement of reduced (GSH) and oxidised (GSSG) glutathione is a standard method to evaluate the antioxidant capacity of cells. The method is based on the reaction of GSH with 5,5'-dithiobis(2-nitrobenzoic acid) (DTNB), whereby 2-nitro-5-mercaptobenzoic acid is cleaved, which then leads to a color change. The frozen liver samples were homogenised in a 0.2 M sodium phosphate buffer (pH 8), containing 0.005 M EDTA, and four volumes of 25 % metaphosphoric acid. Subsequently, the samples were centrifuged at 9000 x rcf for 30 min at 4 °C. The absorbance was read at 412 nm using a Spekol 1100 (Carl Zeiss GmbH, Jena, Germany). A standard curve was performed with GSH.

In addition, the GSSG concentration was assessed fluorometrically (fluorescencespectrophotometer F-2000, Hitachi, Tokio, Japan). This reaction is based on the pH-dependent reaction of GSSG with o-Phthalaldehyde. The fluorescence intensity is proportional to the GSSG concentration. Afterward, the samples were excited at 350 nm and emitted at 30 nm.

### Intravital microscopy (IVM)

IVM was conducted to monitor the mitochondrial membrane potential *in vivo* with confocal laser scanning microscopy. Therefore, the animals remained under deep general anesthesia using isoflurane and pain reflexes were assessed to gauge the depth of anesthesia. The method of monitoring mitochondria by IVM was recently described (Press et al., A new fluorescent dye for cell tracing and mitochondrial imaging in vitro and in vivo. J Biophotonics, Nov 2015.). The hepatic IRI experiments were performed with Rhodamine 123. This is a cationic fluorophore that is taken up by polarized mitochondria in response to their negative membrane potential. A final dilution that contained 250 $\mu$g

in 100 µl was injected into the tail vein 5 min before the onset of the experiment. The liver was exposed and placed on a cover slip. The left lateral lobe was clamped for 1 h and imaged. After the ischemia, the clamped lobe was reperfused for 1 h. Images were acquired using a LSM 780 microscope (Zeiss AG, Jena, Germany). Rhodamine 123 was excited at 488 nm (Argon/2 laser) and the emitted light was detected by a monochromatic filter at $530 \pm 30$ nm (GaAsP detector). To visualise the liver tissue, NADP(H) autofluorescence of hepatocytes was exploited (excitation: $385 \pm 15$ nm, laser-diode; emission: $430 \pm 20$ nm). Images were taken every 10 min during the ischemia. The clamp opening procedure was performed for 3 min with a time series of images every 10 s. Subsequently, the reperfusion was imaged for 1 h every 5 min. This monitoring was carried out with a 20x plan-apochromat (1.0, corrected) to evaluate the different hepatic zones. The standard deviation of the Rhodamine 123 fluorescence signal was calculated to quantify the changes of the mitochondrial granularity over time. Images after hepatic IRI were acquired in the laser-scanning mode using a 63x long distance C-apochromat (NA 1.15, water corrected) for representative images.

Automated image analysis

To characterize the roughness of the mitochondrial network, an automated image analysis procedure was implemented in the programming language "python" (https://www.python.org/). The procedure is based on partitioning of the image into small areas (superpixels) of sizes approximately equal to the size of a hepatocyte and on computing the intensity variation in each of the superpixels, *i.e.* the roughness of the mitochondrial network in hepatocytes is characterized based on the Rhodamine signal. This is done by computing the Sobel edges of the Rhodamine signal averaged over the whole superpixel and by computing the standard deviation of the Rhodamine intensity. The medians of these values are taken to represent the roughness of the mitochondrial network in each image. Details on the image analysis are provided below.

Code availability

The source code for the automated image analysis is available from the authors upon request.

Measurement of mitochondrial respiration in situ

The mitochondrial integrity and function was analysed by measuring the oxygen consumption of the different complexes in the mitochondrial respiration chain. Therefore, the isolation and oxygen consumption of the mitochondria were based on the protocol of Frezza et al. (Organelle isolation: functional mitochondria from mouse liver, muscle and cultured fibroblasts. Nat Protoc, 2 (2): 287-295, 2007.) with some modifications. All chemicals and kits were supplied by Sigma Aldrich, Germany.

The oxygen consumption was measured by a microcathode oxygen electrode (S1782, Strathkelvin Instruments, Great Britain). The electrodes were calibrated according to the manufacturer's protocol. Mitochondria (1 mg protein) were suspended in 1 ml experimental buffer (800 mM KCl, 10 mM Tris/MOPS, 40 mM EGTA/Tris 4, 400 mM $KH_2PO_4$). As a substrate of complexes II, III and IV, 5 mM succinate was added. The main read out is the "respiratory control ratio (RCR)" that can be calculated by the ratio of "state 3" and "state 4" respiration, which is equivalent to the ratio of ADP-dependent respiration and ADP-independent respiration. Therefore, 150 µM ADP was added to the chamber resulting in a further increase in oxygen consumption of the mitochondria. If the ADP was consumed completely, the slope of the oxygen consumption ("state 4" respiration) looked similar to the "state 2" slope. As a positive control, 100 nM carbonyl cyanide 4-(trifluoromethoxy)phenylhydrazone (FCCP) was added at the end of every experiment which lead to the uncoupling of the mitochondria.

Isolated liver perfusion ex vivo

The influence of NaHS and STS on hepatic IRI was determined *ex vivo* by the isolated liver perfusion method. Therefore, a monovascular perfusion of the liver through the portal vein (anterograde perfusion) was carried out. The portal vein was cannulated and perfused with warm, oxygenated Krebs-Henseleit buffer (KBH) (Biochrom, Berlin, Germany). During the whole experiment, the pressure within the liver was monitored via a pressure transducer (Biopac®Systems Inc, MP150, CA, USA) to control the physiological pressure of the liver. In this recirculating system, it was possible to collect the outflow and analyse the LDH level over time. Livers were perfused 5 min one-way and 5 min recirculating to get a stable baseline. Afterward, the pump was stopped for 1 h followed by 1 h reperfusion. During the reperfusion, outflow samples were collected every 10 min. Finally, the liver tissue samples were stored for histological and molecular biological analysis.

LDH Assay

The LDH release during isolated liver perfusion was determined according to the manufacture's protocol of Cytotoxicity Detection KitPLUS (LDH) from Roche, Germany. The formed formazan is water soluble and was detected at 490 nm using the UVTransilluminator E.A.S.Y. RH (Herolab, Germany).

Western blotting

The total protein content was isolated from the frozen tissue samples by an protein isolation buffer pH 7.5 (10 mM Tris, 250 mM saccharose, 1 mM EDTA, 1 % phosphatase-inhibitor, 1 % protease-inhibitor). To keep organelles and membranes as intact as possible, a tightly fitting Dounce homogenizer (Kimble Chase, Mexico) was used resulting in a mild and mechanical homogenisation. Afterward, the homogenate was centrifuged at 900 x rcf for 10 min at 4 °C. The protein content in the post nucleus supernatant was determined using the BCA assay (Sigma Aldrich, Hamburg, Germany). In addition, the samples were incubated for 15 min at room temperature with Laemmli sample buffer to denature the protein structure.
Afterward, the proteins were separated by size using 4-12 % SDS-gels (NuPAGE® Bis-Tris Gel, Invitrogen, Germany) in a SDS-Gel electrophoresis (XCellSureLock® Mini Gel chamber and NuPAGE®MOPS SDS Running Buffer, Invitrogen, Germany). Subsequently, the proteins were transferred to a PVDF-membrane (Roth, Germany). A blocking solution containing tris-buffered saline and tween 20 (TBST) pH 7.5 (10 mM Tris, 100 mM NaCl, 0.1 % Tween 20) 5 % BSA or skim milk (Sigma-Aldrich, Germany) was used to block unspecific bindings. The membrane was incubated with this solution for 1 h at room temperature. Next, the membrane was incubated with a rabbit polyclonal LC3B antibody (Cell Signaling Technology®, Danvers, USA 1:500 dilution), a rabbit polyclonal TOM20 antibody (Abcam®, Cambridge, UK 1:500 dilution) or a rabbit polyclonal $\beta$-actin antibody (Abcam®, Cambridge, UK 1:2,000 dilution) overnight at 4 °C. All primary antibodies were diluted in the blocking solution depending on the manufacture's protocol. After washing the membrane with TBST buffer 3 times, a horseradish peroxidase (HRP)-conjugated secondary antibody (1:5,000) was added for 1 h at room temperature. In the last step, the membrane was washed again with TBST buffer 3 times.
Detection of the secondary antibody by chemiluminescence was performed using HRP substrate (Immobilon Western HRP Substrat, Merck Millipore, USA) by the LAS3000 (GE Healthcare, Frankfurt, Germany). Finally, the target proteins were quantified by densitometric analysis in the software *ImageJ* (National Institutes of Health, USA). B-actin served as loading control to calculate the differences in the protein expression between the groups.

Immunofluorescence tissue staining

Immunofluorescence staining was used to investigate the TOM20 localisation. In the first step, the slides were permeabilised by a citrate buffer pH 6.1 (Dako; Germany) at 100 °C for 20 min. After cooling down and washing (TBS with 0.5 % triton-X), unspecific bindings were blocked by a blocking solution (TBS with 5 % BSA) for 1 h at 37 °C. Furthermore, the slides were incubated with a rabbit polyclonal TOM20 antibody (Abcam®, Cambridge, UK 1:500 dilution) over night at 4 °C. The negative controls were only incubated with washing buffer. After further washing steps, the slides were incubated with fluorescence marked antibody, Cy™5 Donkey Anti-Rabbit IgG 1:1,000 Jackson Immuno Research, USA overnight at 4 °C. After last step of washing, the slides were mounted with a DAPI-containing mounting medium (Dako, Germany).
The slides were imaged by the confocal LSM 780 (Zeiss, Jena, Germany). TOM20 was excited at 633 nm (Argon/2 laser) and the emitted light was detected by a monochromatic filter 640-730 nm (GaAsP detector). The DAPI staining of the nuclei was visualised by an excitation of $405 \pm 15$ nm and the emission was detected at 410-530 nm. The positive stained cells were automatically quantified using the *ImageJ* software (National Institute of Health, USA). A threshold was set for the DAPI and the TOM20 signal. Afterward, the cells and nuclei were quantified by the particle analyser within the software.

Hematoxylin and eosin (HE) staining

Hematoxylin and eosin staining is the gold standard to verify pathomorphological changes in the tissue after IRI. Hematoxylin has a deep blue-purple color and stains nucleic acids, whereas eosin is pink and stains proteins nonspecifically. The slides were deparaffinated and stained for 2 min in hematoxylin (P. Mayer, Sigma-Aldrich, Germany). After a washing step, the slides were stained 15 min in eosin (Merck, Darmstadt, Germany). In addition, further washing steps were carried out and the slides were dehydrated in an ascending alcohol series. In the last step, the slides were mounted with the Entellan®-mounting medium (Merck, Darmstadt, Germany). Finally, the slides were scanned with a whole slide scanner (Hamamatsu Nanozoomer, Germany) and imaged by the *NDP.view2* viewing software (Hamamatsu, Germany). Necrosis was expressed as the percentage of necrotic tissue: 0, no necrosis; 1, less than 25 %; 2, 25 %-50

%; 3, 50 %-75 %; and 4, at least 75 % necrosis. The necrotic areas are characterised by cell swelling and cell membrane rupture as well as a fragmentation of the nucleus. All histological analyses were performed in a blinded fashion with respect to the experimental groups.

Transmission electron microscopy (TEM)

The analysis of the ultra-structure of the tissue was performed by TEM. Therefore, the liver had to be perfused with 4 ml/min for 2 min with warm, oxygenated KBH to wash out all blood as described in section 3.4. Afterward, the liver was perfused with fixation buffer pH 7.4 (4 % paraformaldehyde, 2.5 % glutaraldehyde, 0.1 M sodium cacodylate) for 4 min. After the fixation, the liver was removed and cut into 1-2 mm slices. These slides were incubated overnight in the fixation buffer at 4 $\circ$C. Subsequently, the slices were cut into cubes and stored in 0.1 M cacodylate buffer.
The samples were embedded in Araldite resin (Plano, Wetzlar, Germany) according to manufacturer's instruction. Semithin sections (1 $\mu$m) were stained with Richardson's methylene blue to get a general overview. The areas of the samples were then further trimmed down to 500 x 500 $\mu$m. Ultra-thin sections of 80 nm thickness were cut using the ultramicrotome Ultracut E (Reichert-Jung, Wien, Austria). Finally, sections were investigated using a transmission electron microscope EM900 (Zeiss).

Statistical analysis

All data in this study are presented as mean $\pm$ standard error of the mean (SEM). Differences in data between groups were compared using Prism 6 software (Graph-Pad Prism®, USA) with the Kruskal-Wallis test when comparing three or more groups. If a significant difference was found with the Kruskal-Wallis test, then Dunn's multiple-comparison test was used for post hoc analysis. When two or more groups were compared with a control group, a two way ANOVA with Sidak's multiple comparison test was carried out. If three or more distinct groups were compared at different time points, a two-way ANOVA with Tukey's multiple comparison was used.

**Automated image analysis**

To characterize the roughness of the mitochondrial network in hepatocytes from confocal microscopy images of different experimental conditions, we developed an automated image analysis procedure was developed (see Figure 12). The procedure was implemented in the programming language "python" (https://www.python.org/) and included the following steps:

1. Normalization of the Rhodamine channel $R(x,y)$ in each image:

$$N(x,y) \ = \ \frac{R(x,y)}{\widetilde{R(x,y)}},$$

where $N(x,y)$ corresponds to the normalized Rhodamine signal relative to the mean value $\widetilde{R(x,y)}$ of the Rhodamine signal. To prevent amplification of noise in images with insufficient signal, images with the maximum of $R(x,y)$ lower that 10 (out of 255) were excluded from the further analysis.
2. Detection of vessels $V(x,y)$:

2.1. Normalization of $N(x,y)$ to its 95-th percentile $p_{95}$:

$$N_v(x,y) = \frac{N(x,y)}{p_{95}\{N(x,y)\}} \cdot 255 \,.$$

2.2. Computation of $G(x,y)$ as a Gaussian filter of $N_v(x,y)$ with standard deviation $\sigma = 1$ $\mu m$ to smooth intensity variations at this scale.
2.3. Thresholding of $G(x,y)$ to detect vessels:

$$T(x,y) = \begin{cases} 255, & if\ G(x,y) > t, \\ 0, & otherwise. \end{cases}$$

Here, $t = 50$ was used, which - by visual inspection - gave the most realistic representation of the vessels.

2.4. Computation of $T'(x, y)$ as morphological closing of $T(x,y)$ to reduce noise and/or small objects in the foreground (tissue).

2.5. Computation of $T''(x, y)$ as morphological opening of $T'(x, y)$ to reduce noise and/or small objects in the background (vessels).

2.6. Computation of vessels mask $V(x,y)$ by inversion of $T''(x,y)$:

$$V(x,y) = \begin{cases} 255, & if\ T''(x,y) = 0, \\ 0, & if\ T''(x,y) = 255. \end{cases}$$

3. Segmentation of cells $C(x,y)$:

3.1. Normalization of $N(x,y)$ to its maximum value:

$$N_c(x,y) = \frac{N(x,y)}{max\ \{N(x,y)\}} \cdot 255.$$

3.2. Computation of superpixels $S(x,y)$ from $N_c(x,y)$ by application of the SLIC algorithm [1]. The following parameters for the SLIC algorithm were used to achieve a reasonable segmentation result as confirmed by visual inspection:

3.2.1. Number of superpixels is taken such that the average superpixel size is 900 $\mu m^2$, which corresponds to a 30x30 $\mu$m square, representing the approximate size of a hepatocyte.

3.2.2. Compactness: 40

3.2.3. Smoothing sigma: 0.5 $\mu$m

3.2.4. Enforce connectivity: True

3.3. For each superpixel $S(x,y)$, its total area ($A_s$) and the area covered by vessels ($A_v$) are computed.

3.4. Removal of all superpixels with $\frac{A_v}{A_s} > 0.4.$ Labeling of remaining superpixels as $C(x,y)$.

4. Classification of cells into bright and dark regions $R(x,y)$

4.1. For each cell $C(x,y)$, $I(n) = \widetilde{N(x,y)}$ is computed, where $\widetilde{N(x,y)}$ is the mean intensity of the normalized Rhodamine channel in a superpixel $n$; $n = [1..N]$, $N$ - the total number of superpixels.

4.2. Collecting of $I(n)$ for all cells of all time points of all control animals. Classification of the cells into 2 classes with respect to $I(n)$ by k-means clustering.

4.3. Computation of corresponding k-means centers: $I_1$ for the dark region, $I_2$ for the bright region.

4.4. Computation of $I(n)$ for all cells in all images (also for those that were not used to compute k-means centers). Assigning all cells to the closest k-means center:

$$R(x,y) = f(x) = \begin{cases} 0, & |I(n) - I_1| \le |I(n) - I_2| \\ 1, & |I(n) - I_2| < |I(n) - I_1| \end{cases}$$

4.5. Assigning to the background all cells $C(x,y)$ that are not in the bright region:

$$C_b(x,y) = \begin{cases} C(x,y), & R(x,y) = 1 \\ 0, & R(x,y) = 0 \end{cases}$$

5. Characterization of the roughness of the mitochondrial network

5.1. Computation of Sobel edges of $N(x,y)$: $S(x,y)$

5.2. In each cell $C_b(x,y)$, computing the mean of the Sobel edges $R_1 = \widetilde{S(x,y)}$ and standard deviation of Rhodamine signal $R_2 = std\{N(x,y)\}$.

5.3. Computing the medians of $R_1$ and $R_2$ over all cells in each image to characterize the roughness of the mitochondrial network in the image. Because the intensity variations averaged by such a statistical approach do not depend on exact boundaries of superpixels, the precise correspondence between superpixels and cells is actually of minor importance.

**Claims**

1. A compound for use in a method for treating hepatic ischemia/reperfusion injury, wherein said compound is a thiosulfate.

2. The compound for use in a method for treating hepatic ischemia/reperfusion injury according to claim 1, wherein said compound is sodium thiosulfate.

3. The compound for use in a method for treating hepatic ischemia/reperfusion injury according to claim 1 or 2, comprising the dosage regimen:

   a) administering to a patient an initial dose of said compound on the first day of treatment.

4. The compound for use in a method for treating hepatic ischemia/reperfusion injury according to claim 3, wherein said initial dose is 0.1 mg/kg body weight to 500 mg/kg body weight of said compound.

5. The compound for use in a method for treating hepatic ischemia/reperfusion injury according to claim 3 or 4, wherein said initial dose is administered before, during or after a liver transplantation.

6. The compound for use in a method for treating hepatic ischemia/reperfusion injury according to claim 5, wherein said transplanted liver originates from a marginal donor or is a fatty liver.

7. A pharmaceutical composition for use in a method for treating hepatic ischemia/reperfusion injury comprising a compound according to claim 1 or 2.

8. A dosage form for use in a method for treating hepatic ischemia/reperfusion injury comprising a compound according to claim 1 or 2.

9. The dosage form for use in a method for treating hepatic ischemia/reperfusion injury according to claim 8, wherein said compound is applied as an oral formulation, injection form or intravenous form.

10. The dosage form for use in a method for treating hepatic ischemia/reperfusion injury according to claim 8 or 9, wherein said dosage form comprises 0.1 mg/kg body weight to 500 mg/kg body weight of a compound according to claim 1 or 2.

**Patentansprüche**

1. Eine Verbindung zur Verwendung in einem Verfahren zur Behandlung einer hepatischen Ischämie/Reperfusionsschaden, wobei die Verbindung ein Thiosulfat ist.

2. Die Verbindung zur Verwendung in einem Verfahren zur Behandlung von hepatischer Ischämie/Reperfusionsschaden gemäß Anspruch 1, wobei die Verbindung Natriumthiosulfat ist.

3. Die Verbindung zur Verwendung in einem Verfahren zur Behandlung von hepatischer Ischämie/Reperfusionsschaden gemäß Anspruch 1 oder 2, umfassend das Dosierungsschema:

   a) Verabreichen einer Anfangsdosis der Verbindung an einen Patienten am ersten Tag der Behandlung.

4. Die Verbindung zur Verwendung in einem Verfahren zur Behandlung einer hepatischen Ischämie/Reperfusionsschaden gemäß Anspruch 3, wobei die Anfangsdosis 0,1 mg/kg Körpergewicht bis 500 mg/kg Körpergewicht der Verbindung beträgt.

5. Die Verbindung zur Verwendung in einem Verfahren zur Behandlung von hepatischer Ischämie/Reperfusionsschaden gemäß Anspruch 3 oder 4, wobei die Anfangsdosis vor, während oder nach einer Lebertransplantation verabreicht wird.

6. Die Verbindung zur Verwendung in einem Verfahren zur Behandlung von hepatischer Ischämie/Reperfusionsschaden gemäß Anspruch 5, wobei die transplantierte Leber von einem marginalen Spender stammt oder eine Fettleber ist.

7. Eine pharmazeutische Zusammensetzung zur Verwendung in einem Verfahren zur Behandlung einer hepatischen Ischämie/Reperfusionsverletzung, umfassend eine Verbindung nach Anspruch 1 oder 2.

8. Eine Dosierungsform zur Verwendung in einem Verfahren zur Behandlung einer hepatischen Ischämie/Reperfusionsverletzung, umfassend eine Verbindung nach Anspruch 1 oder 2.

9. Die Darreichungsform zur Verwendung in einem Verfahren zur Behandlung von hepatischer Ischämie/Reperfusionsschaden gemäß Anspruch 8, wobei die Verbindung als orale Formulierung, Injektionsform oder intravenöse Form angewendet wird.

10. Die Darreichungsform zur Verwendung in einem Verfahren zur Behandlung von hepatischer Ischämie/Reperfusionsschaden gemäß Anspruch 8 oder 9, wobei die Darreichungsform 0,1 mg pro kg Körpergewicht bis 500 mg pro kg Körpergewicht einer Verbindung nach Anspruch 1 oder 2 umfasst.


**Revendications**

1. Composé destiné à être utilisé dans un procédé de traitement d'ischémie/lésion de reperfusion hépatique, dans lequel ledit composé est un thiosulfate.

2. Composé destiné à être utilisé dans un procédé de traitement d'ischémie/lésion de reperfusion hépatique selon la revendication 1, dans lequel ledit composé est le thiosulfate sodique.

3. Composé destiné à être utilisé dans un procédé de traitement d'ischémie/lésion de reperfusion hépatique selon la revendication 1 ou 2, comprenant le régime posologique :

   a) administrer à un patient une dose initiale dudit composé le premier jour de traitement.

4. Composé destiné à être utilisé dans un procédé de traitement d'ischémie/lésion de reperfusion hépatique selon la revendication 3, dans lequel ladite dose initiale est de 0,1 mg/kg de poids corporel à 500 mg/kg de poids corporel dudit composé.

5. Composé destiné à être utilisé dans un procédé de traitement d'ischémie/lésion de reperfusion hépatique selon la revendication 3 ou 4, dans lequel ladite dose initiale est administrée avant, pendant ou après une greffe de foie.

6. Composé destiné à être utilisé dans un procédé de traitement d'ischémie/lésion de reperfusion hépatique selon la revendication 5, dans lequel ledit foie greffé provient d'un donneur marginal ou est une stéatose hépatique.

7. Composition pharmaceutique destinée à être utilisée dans un procédé de traitement d'ischémie/lésion de reperfusion hépatique comprenant un composé selon la revendication 1 ou 2.

8. Forme de dosage destinée à être utilisée dans un procédé de traitement d'ischémie/lésion de reperfusion hépatique comprenant un composé selon la revendication 1 ou 2.

9. Forme de dosage destinée à être utilisée dans un procédé de traitement d'ischémie/lésion de reperfusion hépatique selon la revendication 8, dans laquelle ledit composé est appliqué en tant que formulation orale, forme d'injection ou forme intraveineuse.

10. Forme de dosage destinée à être utilisée dans un procédé de traitement d'ischémie/lésion de reperfusion hépatique selon la revendication 8 ou 9, dans laquelle ladite forme de dosage comprend 0,1 mg/kg de poids corporel à 500 mg/kg de poids corporel d'un composé selon la revendication 1 ou 2.

Fig. 1

Fig. 2

Fig. 3

Fig. 4

Fig. 5

Fig. 6a

Fig. 6b

Fig. 7

Fig. 8

Fig. 9

Fig. 10

control, w/o STS, STS preconditioning, STS post treatment

Fig. 11

Fig. 12

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2008043081 A

**Non-patent literature cited in the description**

- *CHEMICAL ABSTRACTS,* 7772-98-7
- *CHEMICAL ABSTRACTS,* 101202-17-7
- **YAO et al.** Establishment of a rat model of portal vein ligation combined with in situ splitting. *PLoS One,* 2014, vol. 9 (8), e105511
- **WANG et al.** H2S as a physiologic vasorelaxant: hypertension in mice with deletion of cystathionine gamma-lyase. *Science,* October 2008, vol. 322 (5901), 587-590
- **PRESS et al.** A new fluorescent dye for cell tracing and mitochondrial imaging in vitro and in vivo. *J Biophotonics,* November 2015
- **FREZZA et al.** Organelle isolation: functional mitochondria from mouse liver, muscle and cultured fibroblasts. *Nat Protoc,* 2007, vol. 2 (2), 287-295